# EUROPEAN PATENT SPECIFICATION

(11) **EP 3 436 154 B1**
(45) Date of publication and mention of the grant of the patent: **16.11.2022**
(21) Application number: 17714554.7
(22) Date of filing: 27.03.2017
(51) Int. Cl.: A61Q 3/00, A61Q 3/02, A61K 8/92, A61K 8/97, A61K 8/06

(54) **NAIL COMPOSITION**
ZUSAMMENSETZUNG FÜR NÄGEL
COMPOSITION POUR DES ONGLES

(30) Priority: 29.03.2016 GB 201605257
(43) Date of publication of application: 06.02.2019
(73) Proprietor: Slixir Ltd, Castleford, Yorkshire WF10 2AT (GB)
(72) Inventor: GILMORE, Helen, Castleford Yorkshire WF10 2AT (GB)
(74) Representative: Secerna LLP
(86) International application number: PCT/GB2017/050857
(87) International publication number: WO 2017/168136

(56) References cited:
- DE-A1-102011 103 882
- US-A1- 2014 261 512
- Anonymous: "GNPD - Purfection Hand Cream", , 1 July 2010 (2010-07-01), XP055370901, Retrieved from the Internet: URL:http://www.gnpd.com/sinatra/recordpage /1365288/from_search/GYh4LwwDuN/?page=1 [retrieved on 2017-05-09]
- Anonymous: "GNPD - Seconds Hand Cream", , 1 February 2016 (2016-02-01), XP055370905, Retrieved from the Internet: URL:http://www.gnpd.com/sinatra/recordpage /3749853/from_search/rR10DMLePW/?page=1 [retrieved on 2017-05-09]
- Anonymous: "GNPD - Hydravest Intense Moisturizing Hand & Nail Cream", , 1 October 2015 (2015-10-01), XP055370913, Retrieved from the Internet: URL:http://www.gnpd.com/sinatra/recordpage /3512197/from_search/cWo6EKp9cR/?page=1 [retrieved on 2017-05-09]
- Joni Loughran: "Natural Ingredients for Skin Care" In: "Natural Skin Care: Alternative and Traditional Techniques", 1 January 2002 (2002-01-01), B. Jain Publishers (P) Ltd., New Delhi, India, XP055281927, ISBN: 978-81-70-21995-8 pages 101-110, pages 106, 107

## Description

### Field of the Invention

Certain aspects of the present invention relate to a composition for application to a cured, applied polymeric nail coating, hands and cuticles. Particularly, although not exclusively certain embodiments of the present invention relate to a composition which acts to prolong duration and/or maintain the appearance of an applied nail coating applied to a nail. Aptly, the composition may act as a post-application plasticizer of the nail coating.

### Background to the Invention

Nail polish wearers traditionally seek to extend the wear of their nail coating for as long as possible. Currently, it has been the nail coating itself, top and basecoats which have been marketed to consumers as promising increased longevity and effective manicure maintenance.

Base and top coats, proper nail preparation (to avoid chipping or flaking of the natural nail beneath) and advancements in formulation are all know as ways to improve the life of nail coatings. Longer-wear compositions such as so-called "gel polish" became available in the UK around 2005. These compositions are a hybrid between traditional nail polish compositions and more invasive acrylic or gel nail 'extension' services. They typically offer up to a two week nail coating life span. Typically the gel polish compositions are photo-crosslinkable and therefore can be cured under UV or LED light. US 2014/261512 A1 relates to a composition which acts to prolong duration and/or maintain the appearance of an applied nail coating applied to a nail. However, the prior art document is silent to a cream or emulsion and contains no anitinflammatory agents.

Use has already been made in polish formulation of plant oils as plasticizers in a formula. As the nail coating ages, the plasticizers (whether they be plant or other chemicals such as dibutyl phthalate) and solvents are lost to evaporation and contact with other external chemicals such as household cleaning products and soaps. The loss of plasticizer is often accelerated by decomposition of nitrocellulose which produces nitrous oxides which in turn combine with water to form nitrous and nitric acids. Such acids typically affect the stability of plasticizers and their interaction with the polymeric material of the nail coating, leading to premature degradation of the coating.

It is an aim of certain embodiments of the present invention to at least partially mitigate the problems associated with the prior art.

It is an aim of certain embodiments of the present invention to provide a composition which acts to continue to plasticize and therefore prolong the duration and/or appearance of a nail product which is applied to a person's nail.

### Description of Certain Embodiments of the Invention

In a first aspect, there is provided a composition, the composition being for application to an applied, cured nail coating, and wherein the composition comprises at least one aqueous phase and at least one oily phase, the oily phase comprising at least one wax ester and at least one plant oil component having a positive ionic charge, wherein the composition is in the form of a cream, wherein the at least one plant oil component is avocado oil and the at least one wax ester is a jojoba oil wax ester and further wherein the cream composition comprises at least one anti-inflammatory agent, wherein the at least one anti-inflammatory agent is selected from a silver-containing component, a zinc containing component and a copper containing component.

In certain embodiments, the composition is considered to mimic the effects of the original plasticizer present in the applied formula. Furthermore, it is considered that the composition acts to replace the original plasticizer which is lost to solvent evaporation and water transpiration. In certain embodiments, the plant oils in the composition render the nail coating more hydrophobic, effectively creating an occlusive layer which slows down solvent evaporation, helps to prevent water absorption and promotes flexibility.

Aptly, the composition is in the form of an oil-in-water composition.

Aptly, the composition is a cream. As used herein, the term "cream" refers to a composition which has one or more of the following characteristics:
a. Forms a stiff peak (as opposed to a soft peak or no peak, as a lotion would be);
b. Opaque;
c. Dries almost immediately on application;
d. Semi-solid;
e. Non-pourable;
f. High viscosity; and
g. Low loss of mass on drying (approximately 30-40% loss of mass as compared to the initial mass of the composition on application).

In certain embodiments, the composition comprises greater than 20% w/w water and volatile components and less than 50% w/w hydrocarbons, waxes or polyols.

In certain embodiments, the at least one plant oil component comprises a fatty acid selected from oleic acid, palmitic acid, linoleic acid and/or combinations thereof.

Aptly, the at least one plant oil component is avocado oil.

In certain embodiments, the composition comprises the at least one plant oil component in a concentration of between 0.1 and 2.5% w/w.

In certain embodiments, the composition comprises the at least one plant oil component in a concentration of between 0.1 and 0.5% w/w.

In certain embodiments, the composition comprises the at least one plant oil component in a concentration of between 0.1 and 0.3% w/w. e.g. 0.2% w/w.

Aptly, the at least one wax ester is a jojoba oil wax ester. The composition may comprise jojoba oil and/or a hydrogenated jojoba oil. In certain embodiments, the wax ester in a concentration of between 1% and 3% w/w of the total weight of the composition Aptly, wax esters are esters of long-chain fatty acids and long-chain fatty alcohols. Aptly, the jojoba wax ester comprises long chain fatty alcohols esterified with long chain (C35 to C46) fatty acids.

In certain embodiments, the composition does not comprise an animal or mineral oil, or wax ester thereof.

In certain embodiments, the aqueous phase comprises deionised water, and wherein optionally the deionised water is provided in a concentration of between 50% and 75% w/w of the composition.

In certain embodiments, the composition further comprises at least one emulsifier. Aptly, the emulsifier is a non-ionic emulsifier, wherein optionally the non-ionic emulsifier comprises glyceryl stearate and PEG-100 stearate. Aptly, the composition comprises the emulsifier in a concentration of between 1% and 5% w/w. Aptly, the emulsifier is provided in a concentration of 2% w/w.

In certain embodiments, the composition further comprises a perfume.

In certain embodiments, the composition further comprises a texturizing agent. In certain embodiments, the texturizing agent comprises a plurality of particles.

In certain embodiments, the composition further comprises at least one preservative.

In certain embodiments, the composition does not contain an alcohol.

In certain embodiments, the composition further comprises at least one anti-inflammatory agent. In certain embodiments, the anti-inflammatory agent is a metal component. Aptly, the metal component is selected from a silver-containing component, a zinc containing component and a copper containing component. Aptly, the copper containing component is a copper complex.

In certain embodiments, the composition comprises a plurality of anti-inflammatory agents. Aptly, the composition further comprises colloidal oat flour.

In certain embodiments, the composition comprises one or more active agents such as for example an anti-inflammatory agent. Anti-inflammatory agents may be used to heal damaged nail cuticles and/or skin surrounding the nail.

In certain embodiments, the anti-inflammatory agent is a metal compound such as for example a compound comprising silver, zinc and/or copper. In certain embodiments, the composition comprises a copper compound e.g. Acqua-Biomin Copper Y3 PF (available from Lonza). Copper is an anti-inflammatory and therefore in certain embodiments the composition may be used to heal damaged cuticles or other skin surrounding the nail.

In certain embodiments, the composition further comprises colloidal oat flour. Aptly, the colloidal oat flour acts as an anti-inflammatory and antioxidant. Aptly, the colloidal oat flour comprises one or more of beta-glucans, phenols, starch, savenacins, saponins, flavonoids and Vitamin E.

In certain embodiments, the composition further comprises a chelating agent, wherein optionally the chelating agent is EDTA or a salt thereof.

In certain embodiments, the composition further comprises at least one emollient.

In certain embodiments, the composition does not comprise hydrophobic silica airgel particles, such as aerogel silylated silica particles,

In certain embodiments, the composition consists essentially of,
at least one aqueous phase consisting essentially of, deionised water,
at least one oily phase consisting essentially of avocado oil and a jojoba oil wax ester;
EDTA or a salt thereof;
a non-ionic emulsifier;
at least one copper compound;
at least one preservative;
a texturizing agent; and
colloidal oat flour.

Aptly, the nail coating is applied to a nail. Aptly, the nail may be a person's nail and/or a false nail which it itself has been adhered to a person's nail. Aptly, the nail coating is cured either by ambient heat or artificial UV light after being applied to a nail.

In certain embodiments, the composition maintains and/or enhances the visual appearance of the nail coating.

In certain embodiments, the composition is adapted to prevent and/or reduce evaporation of solvents from already applied nail coatings.

In certain embodiments, the composition is adapted to replace plasticizing oils in already cured nail coatings.

In certain embodiments, the composition is adapted to reduce and/or prevent brittleness in nail coatings. Brittleness is considered to be one of the principal causes of nail coating degradation and therefore the composition may have utility in preventing and/or reducing degradation of a nail coating, e.g. a nail coating which is applied to a nail.

In certain embodiments, the composition may extend the duration that the nail coating is adhered to a person's nail. Aptly, the duration is extended as compared to the duration the nail coating is adhered to a human's nail without application of the composition described herein.

In a further aspect of the present invention, there is provided a method of extending a duration of adherence of a nail coating to a nail, comprising the step of applying a composition as described herein to the nail coating. Aptly, the nail may be a human's nail and/or a false nail which has itself has been adhered to a person's nail.

In certain embodiments, the nail coating is a nail varnish. Nail varnish is typically based on nitrocellulose which is dissolved in an organic solvent.

In certain embodiments, the nail coating is an acrylic-based composition. In certain embodiments, the nail coating is a gel nail coating. In certain embodiments, the nail coating is a photo-polymerizable and/or photo-crosslinkable composition. Such compositions are described in, for example, US7375144 and WO2012/130604.

In certain embodiments, the nail coating comprises a compound provided in one or more of the following compound classes:
1. Acrylate;
2. Methacrylate;
3. Cyanoacrylate;
4. Urethane Acrylate; and
5. Urethane Methacrylate.

Without being bound by theory, it is considered that the composition of certain embodiments contains a mix of plant oils, some of which are thought to lie adjacent to the upper surface of the nail coating and form an occlusive layer. This adsorbic effect is a result of the ionic bond which is thought to be present between the positive charge of avocado oil and the negative charge of an acrylic polymer which is typically comprised in the nail coating. The non occlusive jojoba oil (a long chain linear ester) provided in the composition is thought to penetrate the nail coating.

It is considered that this dual plant oil action may have a synergistic effect which protects the coating from the evaporation of solvents and water, prevents the penetration of oxidising agents such as UV light and replaces lost plasticizers from the nail coating.

Aptly the composition further comprises a cosmetically acceptable carrier. In certain embodiments, the cosmetically acceptable carrier is in the form of a cream. Aptly, the composition is a fluid e.g. a cream which has a consistency which enables the composition to be easily and quickly massaged into the nail coating and which does not slide or rub off the upper surface of the nail coating easily.

### Description of the Figures

Certain embodiments are described in more detail by way of example only with reference to the accompanying figures in which:

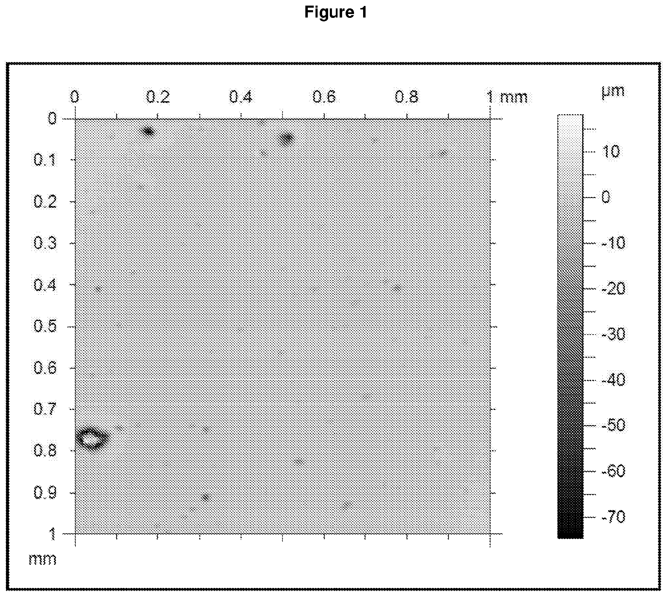

Figure 1 is an SEM image of a glass slide which has been coated with Shellac^{®} and a composition according to Example 1 has been applied on day 0;

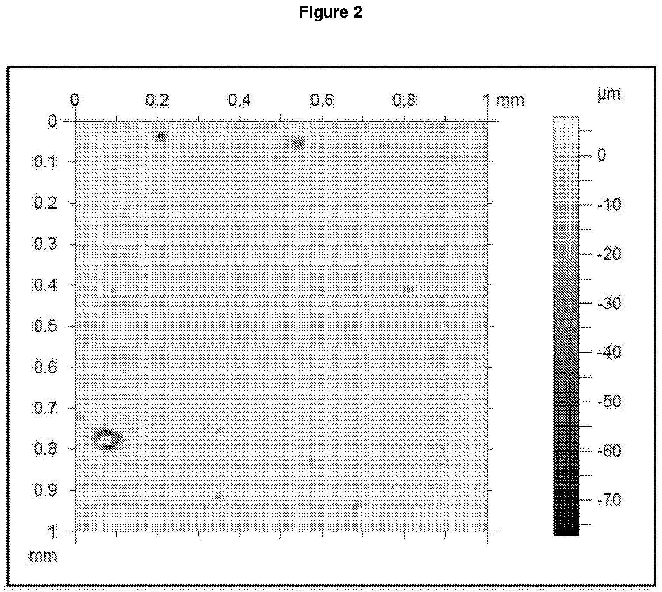

Figure 2 is an SEM image of a glass slide which has been coated with Shellac^{®} and a composition according to Example 1 has been applied on day 1 post- coating;

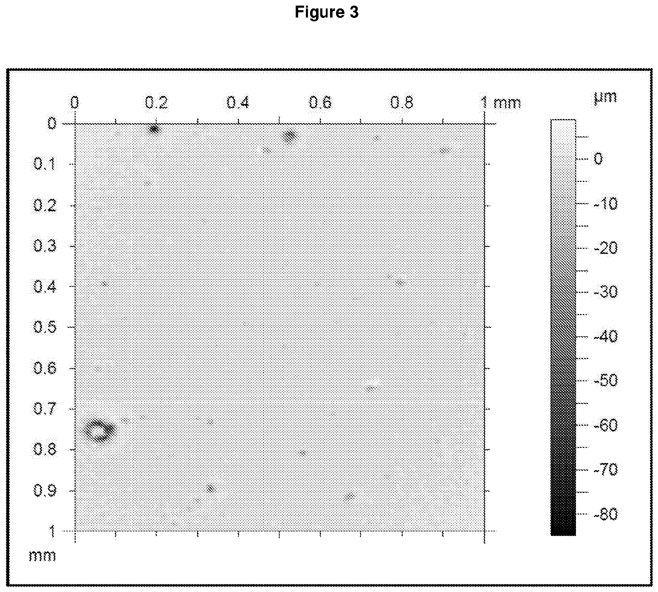

Figure 3 is an SEM image of a glass slide which has been coated with Shellac^{®} and a composition according to Example 1 has been applied on day 3 post-coating; and

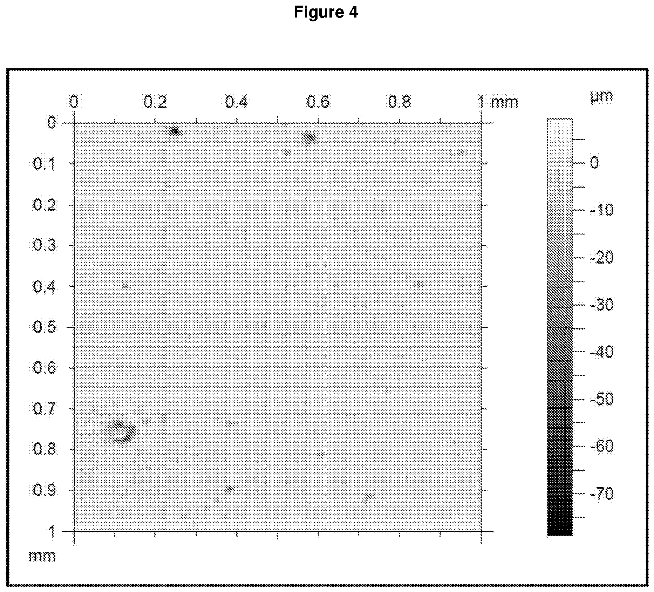

Figure 4 is an image of a glass slide which has been coated with Shellac^{®} and a composition according to Example 1 has been applied on day 5 post-coating.

### Examples

Certain embodiments of the present invention will now be more fully illustrated using the following examples.

### Example 1

The following cream composition was prepared using the method described below.

| **Material Trade Name** | **RM%** |
|---|---|
| Deionised Water | 66.45 |
| Citric Acid Monohydrate | 0.05 |
| Versene NA2 Crystals | 0.10 |
| Surfacare ARC165 | 2.00 |
| Euxyl PE9010 | 1.10 |
| Blanova Vitamin E Acetate | 0.10 |
| Xiameter PMX-0245 Cyclopentasiloxane | 6.00 |
| Avocado Oil Refined | 0.20 |
| Lanol 99 | 8.00 |
| Jojoba Oil Refined | 3.00 |
| Montanov 202 | 3.00 |
| lanol P | 5.00 |
| Simulgel EG | 2.50 |
| Sepimat CP 51.00 | |
| Pink Pepper Pod Frag A322904 | 0.50 |
| Acqua-Biomin Copper Y3 PF | 0.50 |
| AC Colloidal Oat Flour (Avena Sativa (Oat) Kernel Flour)*22003 | 0.50 |

All concentration % are w/w.

### Method of Preparation

1. Weigh out water and heat to 75°C. Then add Versene NA2 crystals and Sepimat CP5 ("aqueous phase");
2. In a separate vessel heat Montanov 202, lanol P, Surfacare 165 ARC, Lanol 99, jojoba oil, blanova vitamin E and avocado oil ("oil phase");
3. Add the oil phase to the aqueous phase and homogenise for 5 minutes;
4. Subsequently add Xiameter PMX 0245 and colloidal oat Flour and Euxyl PE 9010;
5. Cool to 40°C and add with stirring Acqua-Biomin Copper Y3 PF and fragrance and 2/3 of the citric acid. Once fully mixed in then add simulgel EG with slow homogenisation. Fast homogenisation is not used in order to avoid over aeration.
6. When the batch is completely smooth and homogenous, test for pH and Viscosity.

### Example 2

### Analytical Study - The Nano-mechanical effect of plant oils on commercially available nail coatings.

The effect of a composition according to certain embodiments of the present invention on three types of commercially available nail coating was analysed.

### Protocol

A composition was prepared according to Example 1.

Samples of three nail varnish types were prepared on to a glass slide using the following methods;

### Traditional Nail Polish

- Acetone cleansed with cotton wool buds and left to evaporate.
- Shake polish vigorously.
- Apply thin layer to slide.
- Once tacky dry, repeat a second layer.
- Once tacky dry apply a layer of Vinylux.

### Shellac^{®}

- Sic Paper (super fine grade #1000) used to rough the surface of the slide to help varnish adhesion.
- Acetone cleansed with cotton wool buds and left to evaporate.
- Shake base coat polish vigorously.
- Apply thin layer of base coat and cure under UV lamp for 10secs.
- Shake colour coat polish vigorously.
- Apply thin layer of colour coat and cure under UV lamp for 2mins.
- Apply thin layer of colour coat and cure under UV lamp for 2 mins.
- Shake top coat polish vigorously.
- Apply thin layer of top coat and cure under UV lamp for 2mins.
- Wipe top later with cotton wool buds soaked in IPA (Isopropyl Alcohol).

### Removable Gel

Sic Paper (super fine grade #1000) used to rough the surface of the slide to help varnish adhesion.

Acetone cleansed with cotton wool buds and left to evaporate.

Shake base coat polish vigorously.

Apply thin layer of base coat and cure under UV lamp for 10secs.

Using a No6 Flat brush removable sculpting gel was applied evenly to the slide and cured under UV lamp for 2 mins.

Shake top coat polish vigorously.

Apply thin layer of top coat and cure under UV lamp for 2mins.

Wipe top later with cotton wool buds soaked in IPA.

Samples were placed in separate petri dishes to reduce any dust/contamination coming into contact with the surface. These samples were left for 2 weeks to thoroughly set and cure before analysis.

Samples were then measured using:
- a Nikon SMZ10A Stereomicroscope in co-axial light mode to highlight surface differences and by SEM imaging using a FEI Quanta FEG 250 Environmental SEM.
- The profilometry work was carried out on a Taylor Hobson Talysurf CLI1000 using a CLA-3000 gauge.

The test parameters were as follows:

| | |
|---|---|
| • area scanned: | 1mm x 1mm, across the length in the centre of the specimen |
| • grid size: | 5micron |
| • speed: | 1mm/s |
| • sampling rate: | 200Hz |

### Sample analysis schedule:

- Untreated imaging and surface profiles. Composition application.
- Washing using di-ionised water, 24hour imaging and surface profiles. Composition application.
- Washing using di-ionised water and reapplication of composition.
- Washing using di-ionised water, 72 hour imaging and surface profiles. Composition application.
- Washing using di-ionised water and reapplication of composition.
- Washing using di-ionised water, 120 hour surface profiles. Composition application.
- Washing using di-ionised water and reapplication of composition.
- Washing using di-ionised water, 168 hour surface profiles. Composition application.
- Washing using di-ionised water and reapplication of composition.
- Washing using di-ionised water, 216 hour surface profiles.

### Washing method

Samples were rinsed clean using di-ionised water. As the samples were slightly waterproof, this was difficult to rinse away so samples were wetted with di-ionised water and left for a couple minutes, di-ionised water was then rinsed over the top allowing the cream to come away from the surface. It is likely that residual cream was still present on the surface before surface profile measurements.

### Observations

- The composition is readily applied to the nail coatings with excess cream easy to remove without rubbing or washing.
- The composition remains on the surface after washing and so is considered to have filled cracks and crevices in the nail coatings.

### Surface Profiling

The surface profiling images present an optical representation of the nail coating surfaces.

The key measurement for the surface profiling is the **Sa** reading which is an average surface roughness measurement across the area monitored.

Each scan was levelled to ensure compatible orientation. The surface roughness Sa (average departure from the mean plane) was then calculated for the levelled surface.

Three renderings of the data were presented:
- top down pseudo photo
- top down false colour image
- 3D axonometric view
   ∘ a = 50°
   ∘ β = 60°
   ∘ height amplification = 10%

Another set of images was also produced with the aim of exaggerating the relief of the surface:
• top down pseudo photo with light settings
∘ a = 40
∘ β = 40°
∘ contrast = 8
∘ intensity = 100

• top down false colour image
• 3D axonometric view
∘ a = 50°
∘ β = 60°
∘ height amplification = 30%

| | Traditional - um | Shellac - um | Gel - um |
|---|---|---|---|
| Day 0 | 0.303 | 0.819 | 1.24 |
| Sa 24 hours | 0.303 | 0.731 | 1.19 |
| Sa 72 hours | 0.355 | 0.806 | 3.22 |
| Sa 120 hours | 0.514 | 0.872 | 3.35 |
| Sa 168 hours | 0.493 | 0.754 | 3.17 |
| Sa 216 hours | 1.03 | 1.18 | 3.26 |

The roughness results indicate that the composition appears to interact with the surface of the coating from Day 1 with respect to the Shellac^{®} nail coating and the gel polish and from Day 3 with respect to the nitrocellulose-based nail polish. From about day 7, there was a universal increase in the roughness of the nail coating which suggests that the nail coating has degraded by this point.

In certain embodiments, the process includes multiple applications of the inventive composition to the nail coating.

Throughout the description and claims of this specification, the words "comprise" and "contain" and variations of them mean "including but not limited to" and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

## Claims

1. A composition, the composition being for application to an applied and cured nail coating, and wherein the composition comprises at least one aqueous phase and at least one oily phase, the oily phase comprising at least one wax ester and at least one plant oil component having a positive ionic charge, wherein the composition is in the form of a cream,
wherein the at least one plant oil component is avocado oil and the at least one wax ester is a jojoba oil wax ester and further wherein the cream composition comprises at least one anti-inflammatory agent, wherein the at least one anti-inflammatory agent is selected from a silver-containing component, a zinc containing component and a copper containing component.

2. The composition according to claim 1, wherein the composition is in the form of an oil-in-water composition.

3. The composition according to claim1 or claim 2, which comprises the at least one plant oil component in a concentration of between 0.1 and 0.5% w/w.

4. The composition according to claim 3, which comprises jojoba oil and/or a hydrogenated jojoba oil.

5. The composition according to any preceding claim, which comprises the wax ester in a concentration of between 1% and 3% w/w of the total weight of the composition.

6. The composition according to any preceding claim, wherein the aqueous phase comprises deionised water, and wherein the deionised water is provided in a concentration of between 50% and 75% w/w of the composition.

7. The composition according to any preceding claim, which comprises at least one emulsifier.

8. The composition according to claim 7, wherein the emulsifier is a non-ionic emulsifier.

9. The composition according to claim 7, wherein at least one emulsifier comprises arachidyl alcohol, behenyl alcohol & arachidyl glucoside, wherein optionally the emulsifier is Montanov 202 or wherein the at least one emulsifier comprises glyceryl stearate and PEG-100 stearate, wherein optionally the at least one emulsifier is Surfacare ARC165, wherein optionally the composition comprises a combination of Surfacare ARC165 and Montanov 202.

10. The composition according to any of claims 7 to 9, which comprises a total concentration of emulsifiers of between 3% and 7% w/w, and optionally between 4% and 6% w/w.

11. The composition according to any preceding claim, which further comprises one of more of the following:
a) a perfume;
b) a chelating agent, wherein optionally the chelating agent is EDTA or a salt thereof;
c) at least one emollient; and
d) at least one preservative.

12. The composition according to any preceding claim, wherein the copper containing component is a copper complex.

13. The composition according to any preceding claim, which further comprises colloidal oat flour.

14. Use of a composition according to any preceding claim to reduce and/or prevent brittleness in nail coating and/or extending duration that a nail coating is adhered to a person's nail.

15. A method of extending the duration of adherence of a nail coating, comprising the step of applying a composition according to any of claims 1-13 to the nail coating.

## Patentansprüche

1. Zusammensetzung, wobei die Zusammensetzung zum Auftragen auf eine aufgetragene und gehärtete Nagelbeschichtung bestimmt ist, und wobei die Zusammensetzung mindestens eine wässrige Phase und mindestens eine ölige Phase umfasst, wobei die ölige Phase mindestens einen Wachsester und mindestens eine Pflanzenölkomponente umfasst, die eine positive Ionenladung aufweist, wobei die Zusammensetzung in Form einer Creme vorliegt,
wobei die mindestens eine Pflanzenölkomponente Avocadoöl ist und der mindestens eine Wachsester ein Jojobaöl-Wachsester ist und wobei ferner die Cremezusammensetzung mindestens ein entzündungshemmendes Mittel umfasst, wobei das mindestens eine entzündungshemmende Mittel aus einer silberhaltigen Komponente, einer zinkhaltigen Komponente und einer kupferhaltigen Komponente ausgewählt ist.

2. Zusammensetzung nach Anspruch 1, wobei die Zusammensetzung in Form einer Öl-in-Wasser-Zusammensetzung vorliegt.

3. Zusammensetzung nach Anspruch 1 oder Anspruch 2, die die mindestens eine Pflanzenölkomponente in einer Konzentration zwischen 0,1 und 0,5 Gew.-% umfasst.

4. Zusammensetzung nach Anspruch 3, die Jojobaöl und/oder ein hydriertes Jojobaöl umfasst.

5. Zusammensetzung nach einem vorhergehenden Anspruch, die den Wachsester in einer Konzentration zwischen 1 Gew.-% und 3 Gew.-% des Gesamtgewichts der Zusammensetzung umfasst.

6. Zusammensetzung nach einem vorhergehenden Anspruch, wobei die wässrige Phase entionisiertes Wasser umfasst und wobei das entionisierte Wasser in einer Konzentration zwischen 50 Gew.-% und 75 Gew.-% der Zusammensetzung bereitgestellt wird.

7. Zusammensetzung nach einem vorhergehenden Anspruch, die mindestens einen Emulgator umfasst.

8. Zusammensetzung nach Anspruch 7, wobei der Emulgator ein nichtionischer Emulgator ist.

9. Zusammensetzung nach Anspruch 7, wobei mindestens ein Emulgator Arachidylalkohol, Behenylalkohol und Arachidylglucosid umfasst, wobei optional der Emulgator Montanov 202 ist oder wobei der mindestens eine Emulgator Glycerylstearat und PEG-100-Stearat umfasst, wobei optional der mindestens eine Emulgator Surfacare ARC165 ist, wobei die Zusammensetzung optional eine Kombination aus Surfacare ARC165 und Montanov 202 umfasst.

10. Zusammensetzung nach einem der Ansprüche 7 bis 9, die eine Gesamtkonzentration an Emulgatoren zwischen 3 Gew.-% und 7 Gew.-% und optional zwischen 4 Gew.-% und 6 Gew.-% umfasst.

11. Zusammensetzung nach einem vorhergehenden Anspruch, die ferner eines oder mehrere der Folgenden umfasst:
a) ein Parfüm;
b) einen Chelatbildner, wobei der Chelatbildner optional EDTA oder ein Salz davon ist;
c) mindestens einen Weichmacher; und
d) mindestens ein Konservierungsmittel.

12. Zusammensetzung nach einem vorhergehenden Anspruch, wobei die kupferhaltige Komponente ein Kupferkomplex ist.

13. Zusammensetzung nach einem vorhergehenden Anspruch, die ferner kolloidales Hafermehl umfasst.

14. Verwendung einer Zusammensetzung nach einem vorhergehenden Anspruch, um eine Sprödigkeit in der Nagelbeschichtung zu verringern und/oder zu verhindern und/oder die Dauer zu verlängern, während der eine Nagelbeschichtung auf dem Nagel einer Person haftet.

15. Verfahren zum Verlängern der Haftdauer einer Nagelbeschichtung, umfassend den Schritt eines Auftragens einer Zusammensetzung nach einem der Ansprüche 1-13 auf die Nagelbeschichtung.

## Revendications

1. Composition, la composition étant destinée à être appliquée sur un vernis à ongles appliqué et durci, et la composition comprenant au moins une phase aqueuse et au moins une phase huileuse, la phase huileuse comprenant au moins un ester de cire et au moins un composant d'huile végétale ayant une charge ionique positive, la composition étant sous la forme d'une crème,
l'au moins un composant d'huile végétale étant de l'huile d'avocat et l'au moins un ester de cire étant un ester de cire d'huile de jojoba et en outre la composition de crème comprenant au moins un agent anti-inflammatoire, l'au moins un agent anti-inflammatoire étant choisi parmi un composant contenant de l'argent, un composant contenant du zinc et un composant contenant du cuivre.

2. Composition selon la revendication 1, la composition étant sous la forme d'une composition huile-dans-l'eau.

3. Composition selon la revendication 1 ou la revendication 2, qui comprend l'au moins un composant d'huile végétale à une concentration entre 0,1 et 0,5 % p/p.

4. Composition selon la revendication 3, qui comprend de l'huile de jojoba et/ou une huile de jojoba hydrogénée.

5. Composition selon une quelconque revendication précédente, qui comprend l'ester de cire à une concentration entre 1 % et 3 % p/p du poids total de la composition.

6. Composition selon une quelconque revendication précédente, la phase aqueuse comprenant de l'eau déionisée, et l'eau déionisée étant fournie à une concentration entre 50 % et 75 % p/p de la composition.

7. Composition selon une quelconque revendication précédente, qui comprend au moins un émulsifiant.

8. Composition selon la revendication 7, l'émulsifiant étant un émulsifiant non ionique.

9. Composition selon la revendication 7, au moins un émulsifiant comprenant l'alcool arachidylique, l'alcool béhénylique et le glucoside arachidylique, éventuellement l'émulsifiant étant le Montanov 202 ou l'au moins un émulsifiant comprenant le stéarate de glycéryle et le stéarate de PEG-100, éventuellement l'au moins un émulsifiant étant le Surfacare ARC165, éventuellement la composition comprenant une combinaison de Surfacare ARC165 et de Montanov 202.

10. Composition selon l'une quelconque des revendications 7 à 9, qui comprend une concentration totale en émulsifiants entre 3 % et 7 % p/p, et éventuellement entre 4 % et 6 % p/p.

11. Composition selon une quelconque revendication précédente, qui comprend en outre un ou plusieurs parmi :
a) un parfum ;
b) un agent chélateur, l'agent chélateur étant éventuellement l'EDTA ou un sel de celui-ci ;
c) au moins un émollient ; et
d) au moins un agent de conservation.

12. Composition selon une quelconque revendication précédente, le composant contenant du cuivre étant un complexe de cuivre.

13. Composition selon une quelconque revendication précédente, qui comprend en outre de la farine d'avoine colloïdale.

14. Utilisation d'une composition selon une quelconque revendication précédente pour réduire et/ou prévenir la fragilité du vernis à ongles et/ou prolonger la durée pendant laquelle un vernis à ongles adhère à l'ongle d'une personne.

15. Procédé pour prolonger la durée d'adhérence d'un vernis à ongles, comprenant l'étape d'application d'une composition selon l'une quelconque des revendications 1 à 13 sur le vernis à ongles.
